# EUROPÄISCHE PATENTANMELDUNG

(11) **EP 1 101 437 A1**
(43) Veröffentlichungstag der Anmeldung: **23.05.2001**
(21) Anmeldenummer: 00124669.3
(22) Anmeldetag: 10.11.2000
(51) Int. Cl.: A61B 5/00

(54) **Medizinisches System zur Überwachung von Parametern eines Patienten in häuslicher Umgebung**

(30) Priorität: 17.11.1999 DE 19955212
(71) Anmelder: SIEMENS AKTIENGESELLSCHAFT, 80333 München (DE)
(72) Erfinder: Christ, Tilo, 91058 Erlangen (DE); Schmidt, Volker, Dr., 91054 Erlangen (DE); Schneider, Siegfried, Dr., 91056 Erlangen (DE); Striebel, Werner, 91207 Lauf (DE)

(57) **Zusammenfassung**

Die Erfindung betrifft ein medizinisches System zur Überwachung medizinischer Parameter eines Patienten in häuslicher Umgebung, am Arbeitsplatz, in Alten- und Pflegeheimen mit einer Vorrichtung (2) zur Meßwerterfassung bei dem Patienten, einer Vorrichtung (3 bis 8) zur Übertragung der Meßwerte an eine Systemzentrale (6), mit einer Vorrichtung (24) zur Abfrage der Meßwerte und einer Empfangsvorrichtung (20 bis 22) bei der überwachenden Person, wobei die Systemzentrale (6) einen Speicher (15) für die Meßwerte, eine Auswertevorrichtung (12) für die Meßwerte mit einem Komparator zum Vergleich der Meßwerte mit gespeicherten Sollwerten und mit einer Alarmvorrichtung zur Erzeugung eines Alarmsignals und eine Routingvorrichtung (14) zur Weiterleitung des Alarmsignals an eine Empfangsvorrichtung (3 bis 5, 20 bis 22) der zu alarmierenden Person einer Prozeßkette aufweist.

## Beschreibung

Die Erfindung betrifft ein System zur kontinuierlichen Überwachung medizinischer Parameter eines Patienten in häuslicher Umgebung, am Arbeitsplatz oder in einem Pflegeheim. Typische Anwendungsgebiete für ein derartiges System sind beispielsweise die Einstellungsphase auf neue Medikamente, Entgleisungsphasen bei Zuckerkrankheit (Diabetes Mellitus), Glaukomeinstellung, Einstellung des Bluthochdrucks, Betreuung von Risikoschwangerschaften, Notfallüberwachung beispielsweise bei drohendem Schlaganfall, Herzinfarkt, Kardiologische Überwachung bei Herzrhythmusstörungen oder Embolien oder Überwachung bei Lungenerkrankungen wie Asthma oder COLD.

Bei bekannten Meßverfahren mißt der Patient zu Hause seine Werte und liefert sie beim Arzt beispielsweise papiergebunden, als Fax oder über ein beim Arzt auslesbares elektronisches Gerät ab. Eine Überwachung der Mitarbeit und Zuverlässigkeit des Patienten ist für den Arzt jedoch sehr aufwendig. Es können aber auch ambulante Pflegedienste die Meßwerte beim Patienten zu Hause erfassen, die aber häufig aufgrund von Medienbrüchen, dem Problem der Patientenidentifikation und dem Personalaufwand nicht zum Arzt übertragen werden.Bei Notfällen erfolgt eine Koordination der Notfallkette durch die Feuerwehrleitzentrale.

Die Erfindung geht von der Aufgabe aus, ein medizinisches System der eingangs genannten Art derart auszubilden, daß eine kontinuierliche Überwachung medizinischer Parameter eines Patienten bei räumlicher Trennung von Arzt oder Pflegediensten und Patient ermöglicht wird und eine gezielte Information interessierter Parteien, der Ärzte, Pfleger, Patienten, Kostenträger, Kliniken und/oder Reha, über besondere Ereignisse auch bei Abwesenheit des eigentlichen Empfängers bewirkt.

Die Aufgabe wird bei einem erfindungsgemäßen System mit einer Vorrichtung zur Meßwerterfassung bei dem Patienten, einer Vorrichtung zur Übertragung der Meßwerte an eine Systemzentrale, mit einer Vorrichtung zur Abfrage der Meßwerte und einer Empfangsvorrichtung bei der überwachenden Person gelöst, wobei die Systemzentrale einen Speicher für die Meßwerte, eine Auswertevorrichtung für die Meßwerte mit einem Komparator zum Vergleich der Meßwerte mit gespeicherten Sollwerten und mit einer Alarmvorrichtung zur Erzeugung eines Alarmsignals und eine Routingvorrichtung zur Weiterleitung des Alarmsignals an eine Empfangsvorrichtung der zu alarmierenden Person der Prozeßkette. Ein solches System kommt zum Einsatz, um beispielsweise medizinische Leistungen, die einfache Überwachungsmaßnahmen erfordern, aus dem Krankenhausbereich oder der Arztpraxis herauszulösen und in die Lebensumgebung des Patienten zu verlagern. Insbesondere werden von dem System drei Größen überwacht: Die Patientencompliance, die eigentlichen medizinischen Meßgrößen und die Arztcompliance. Damit können Alarme ausgelöst werden, wenn der Patient nicht oder zu häufig Meßwerte erzeugt und übermittelt, wenn die Meßwerte in einem krankhaften Bereich liegen, und wenn die zu alarmierende Stelle nicht adäquat reagiert.

Erforderliche Sofortmaßnahmen im Krankheitsfalle lassen sich unverzüglich einleiten, wenn die Alarmvorrichtung derart ausgebildet ist, daß sie ein Alarmsignal bei Überschreitung insbesondere bei signifikanter Überschreitung der Meßwerte von gespeicherten Sollwertgrenzen erzeugt, das an eine Empfangsvorrichtung einer Person einer Behandlungskette bzw. Notfallkette geleitet wird.

Erfindungsgemäß kann die Alarmvorrichtung derart ausgebildet sein, daß sie ein Alarmsignal bei Ausbleiben von Meßwerten erzeugt, das an eine Empfangsvorrichtung des Patienten geleitet wird, und/oder daß sie ein Alarmsignal bei Ausbleiben von Reaktionen der Behandlungskette auf besonders gekennzeichnete Werte erzeugt, das an eine Empfangsvorrichtung der Notfallkette geleitet wird.

Es hat sich als vorteilhaft erwiesen, wenn die Routingvorrichtung derart ausgebildet ist, daß das Alarmsignal an eine vorbestimmte, auswählbare Empfangsvorrichtung der Prozeßkette geleitet wird oder daß das Alarmsignal durch automatisches Routing an eine von der Routingvorrichtung unter Berücksichtigung der Verfügbarkeit bestimmte Empfangsvorrichtung der Prozeßkette geleitet wird.

Eine schnelle und effektive Benachrichtigung im Notfall läßt sich erreichen, wenn die Routingvorrichtung ein lernendes Expertensystem aufweist, das die Alarmierung der Prozeßkette, beispielsweise die Verständigung des Hausarztes, Rufen des Notarztes, Organisieren des Transportdienstes und/oder Vorbereitung der Klinik, bewirkt.

Unerwünschte Fehlmeldungen werden reduziert bzw. ausgeschlossen, wenn die Auswertevorrichtung ein lernendes Expertensystem aufweist, das die Meßwerte krankheits- und/oder problemspezifisch anhand von Regelsystemen oder auf Wahrscheinlichkeiten basierend interpretiert, aufgrund deren Ergebnis das Alarmsignal ausgelöst wird.

Die Erfindung ist nachfolgend anhand von in der Zeichnung dargestellten Ausführungsbeispielen näher erläutert. Es zeigen:
- Figur 1: ein erfindungsgemäßes medizinisches System,
- Figur 2: Benutzeroberfläche zur Konfiguration des zentralen Alarmgebers und
- Figur 3: Benutzeroberfläche zur Konfiguration

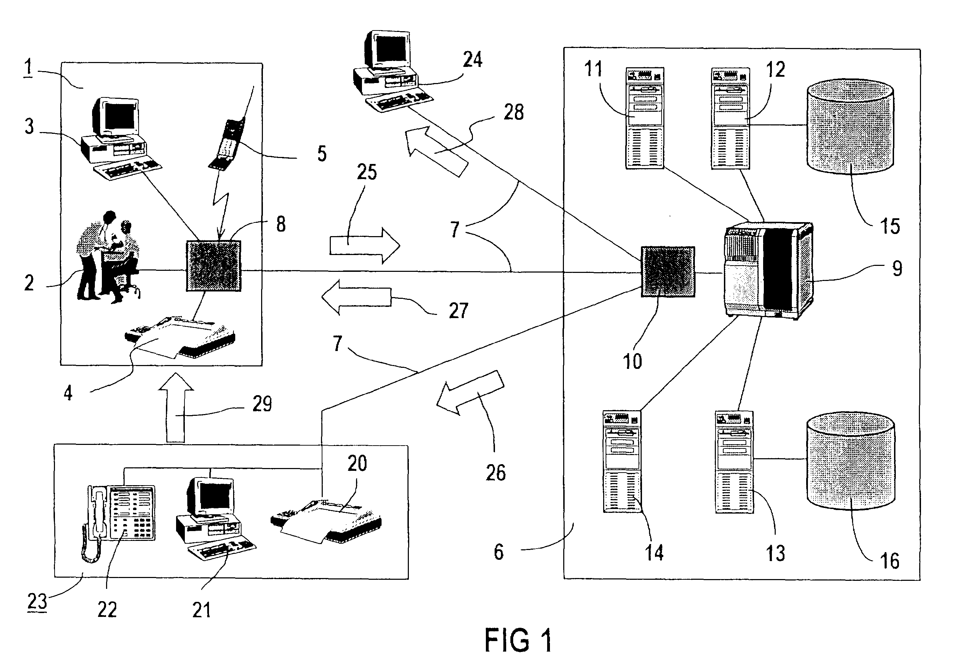

In der Figur 1 ist ein erfindungsgemäßes System zur kontinuierlichen Überwachung medizinischer Parameter eines Patienten in seinem Haus 1 dargestellt, in dem die Meßwerterfassung 2 entweder automatisch oder manuell durch den Patienten oder eine Pflegeperson erfolgt. Diese Meßwerte können direkt per Personal Computer 3, per Faxgerät 4 oder per Telefon oder Handy 5 an eine Systemzentrale 6 weitergeleitet werden. Dies kann beispielsweise über ein ISDN-Netz 7 erfolgen, an das die entsprechenden Endgeräte über ein ISDN-Interface 8 angeschlossen sind.

In der Systemzentrale 6 ist ein Gateway 9 vorgesehen, das über ein ISDN-Interface 10 an dem ISDN-Netz 7 angeschlossen. An dem Gateway 9 kann ein Internet-Proxy-Server 11 zum Zugriff auf das Internet, eine Auswertevorrichtung 12 für die Meßwerte, ein Patientendaten-Server 13 zur Verwaltung der Patientendaten und ein Kommunikations-Server 14 als Routingvorrichtung zur Zusammenarbeit aller Komponenten und Weiterleitung von Meldungen angeschlossen sein. Mit der Auswertevorrichtung 12 ist ein Datenspeicher 15 für die Meßwerte und mit dem Patientendaten-Server 13 eine Datenbank 16 als Speichervorrichtung verbunden.

Die Auswertevorrichtung 12 weist einen Komparator zum Vergleich der Meßwerte mit in dem Datenspeicher 15 gespeicherten Sollwerten und eine Alarmvorrichtung zur Erzeugung eines Alarmsignals bei Überschreitung der Meßwerte von gespeicherten Sollwertgrenzen auf.

An der Systemzentrale 6 sind Empfangsgeräte wie beispielsweise ein Faxgerät 20, Personal Computer 21 oder ein Telefon 22 oder Handy angeschlossen, die zu einer Ärzte-Bereitschaft gehören und beispielsweise in einer Gemeinschaftspraxis 23 von Ärzten angeordnet sein kann.

Weiterhin ist ein Personal Computer 24 einer Praxis eines erstbehandelnden Arztes über das ISDN-Netz 7 mit dem Gateway 9 der Systemzentrale 6 verbunden.

In der Systemzentrale 6 werden die medizinischen Daten über genormte Telekommunikationsschnittstellen eingelesen und in dem Datenspeicher 15 langfristig abgespeichert, die Auswertevorrichtung 12 wertet die medizinischen Daten und den Zeitpunkt der Datenübertragung aus, um Informationsnachrichten versenden zu können. Die Datenübertragung erfolgt beispielsweise über das Internet oder Telefonnetz. Die Vergabe einer Patientenidentifikationsnummer erfolgt über eine Sicherheitsarchitektur.

In der Systemzentrale 6 können alle Informationen zusammengeführt werden, die zur Realisierung der speziellen Ausführungen des Alarmgebers und zur Realisierung einer Weiterleitung von Nachrichten bei Abwesenheit des Empfängers benötigt werden.

Die Endgeräte 2 bis 5 für den Patienten dienen zur Erfassung der medizinischen Daten beim Patienten und Übertragung der Daten an die Systemzentrale 6. Es können von der Meßtechnik beliebige existierende Geräte verwendet werden, beispielsweise EKG-, Blutdruck-, Blutzuckermeßgeräte, Teststreifen, Peak-Flowmeter und/oder Vitalkapazitätsmeßgeräte. Die Datenübergabe 25 der Meßwerte an den Server in der Systemzentrale 6 kann durch das Meßgerät selbst erfolgen, oder über unten beschriebene Eingabemöglichkeiten realisiert werden.

Für die Pfleger ist ein Personal Digital Assistant (PDA) oder ein Laptop vorgesehen, in dem alle Patientendaten eingegeben werden, die von diesem Pflegedienst besucht werden. Die Synchronisierung der Daten kann sofort, beispielsweise per Handy-Internet Verbindung, oder zu einem späteren Zeitpunkt erfolgen, wenn die Schwester wieder im Büro oder Praxis eintrifft.

Die Systemzentrale 6 dient zur Entgegennahme medizinischer Daten über eine der Telekommunikationsschnittstellen und Speicherung in dem Datenspeicher 15. Weiterhin soll sie eine Weiterverarbeitung der medizinischen Daten mit Hilfe der Auswertevorrichtung 12 für die Meßwerte mit der Alarmvorrichtung Alarmvorrichtung und dem Datenspeicher 15 bewirken. Eine Ausgabe der im Datenspeicher 15 gewonnenen Daten an eines der Endgeräte für Ärzte erfolgt über eine Telekommunikationsschnittstelle.

Ein Alarm wird durch die Alarmvorrichtung in der Auswertevorrichtung 12 bei Vorliegen einer der Ereigniskonstellation wie das Ausbleiben von Meßwerten, krankhafte Meßwerte oder Ausbleiben der Reaktion auf generierte Alarme ausgelöst. Dabei wird ein Alarm 26 aufgrund des Abweichens der Meßwerte von den Sollwerten an die Endgeräte 20 bis 22 der Gemeinschaftspraxis 23 weitergeleitet. Ein Alarm 27 an den Patienten wird wegen fehlender Meßwerte ausgelöst. In der Praxis kann der Arzt die in dem Datenspeicher 15 gespeicherten Meßwerte 28 mit dem Personal Computer 24 abgerufen werden.

Die Auswertevorrichtung 12 beurteilt, ob Meßwerte oder Meßwertkonstellationen als krankhaft einzustufen sind. Als Kriterium werden dabei Grenzen aus der Literatur entnommenen, die in dem Datenspeicher 15 abgespeichert sind. Weiterhin können vom behandelnden Arzt für den Patienten individuelle Grenzen definiert werden.

Dazu kann ein Expertensystem eingesetzt werden, das die Meßwerte krankheits- und problemspezifisch anhand von Regelsystemen oder auf Wahrscheinlichkeiten basierend interpretiert. Dabei ist eine Individualisierung des Expertensystems auf den Patienten vorsehbar, d.h. das Expertensystem lernt den Patienten, den es überwacht, im Überwachungsprozeß immer besser kennen, es trifft dazu als lernendes System ständig Prognosen über zu erwartende zukünftige Meßwerte, die es mit den wahren Meßwerten vergleicht. Damit wird eine individualisierte Überwachung realisiert.

Im Fall komplexer Alarme kann ein Expertensystem eingesetzt werden, daß die Prozeßkette, Erinnern des Patienten, Verständigung Hausarzt, Rufen des Notarztes, Organisieren des Transportdienstes und/oder Vorbereitung der Klinik unter Berücksichtigung der Verfügbarkeit der Alarmempfänger übernimmt.

Die Prozeßkette ist dabei die Gesamtheit der beteiligten Personen und Institutionen. Dazu gehören der Patient, die Behandlungskette mit Arzt, Pflege- und Transportdienst sowie die Notfallkette mit Feuerwehrleitstelle, Notfall- und Transportdienste sowie das Krankenhaus.

Alarme können in Abhängigkeit von der Dringlichkeit einer Reaktion in unterschiedlichen Dringlichkeitsstufen generiert werden, beispielsweise dringendst, dringend, Routine oder Standard. Welche Meßwerte zu welchen Dringlichkeitsstufen führen, kann mit den gleichen Mechanismen wie die Beurteilung der Meßwerte festgelegt werden und in dem Datenspeicher 15 abgespeichert sein.

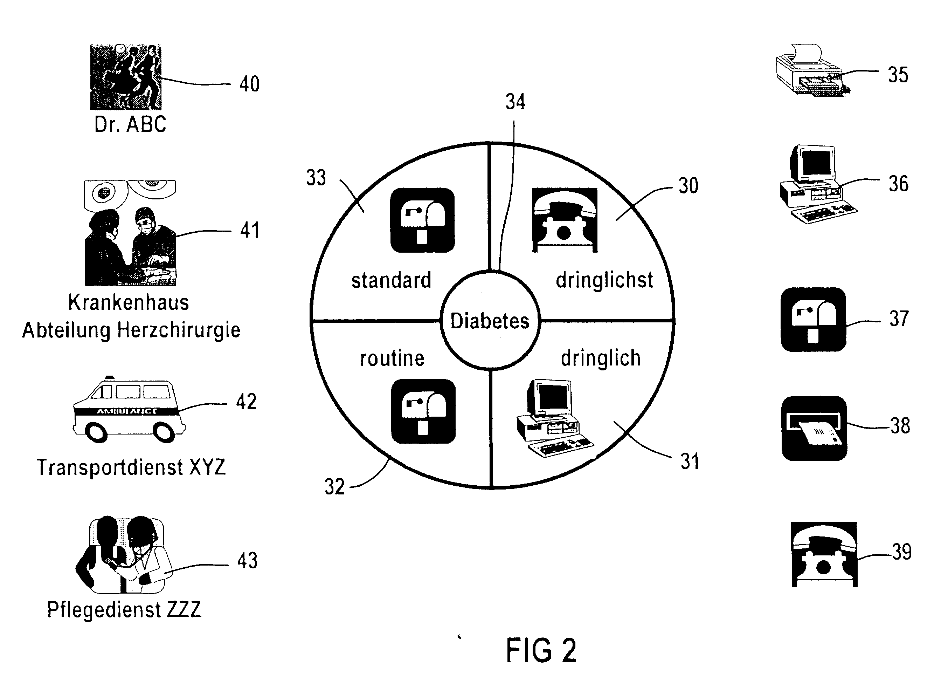

Konfiguration des zentralen Alarmgebers erfolgt durch den Anwender. Die Monitoring Instanz wie der Arzt legt für sich selbst fest, wie er Nachrichten unterschiedlicher Dringlichkeit erhalten will. Dazu verwendet er die in Figur 2 gezeigte Benutzeroberfläche (user interface) . Der Kommunikationskreis ist in Segmente 30 bis 33 unterteilt, die die Dringlichkeitsstufen der Alarmnachrichten repräsentieren. In der Mitte 34 sind die Ausprägungsmöglichkeiten des Kommunikationskreises angegeben. Beim Beispiel gemäß Figur 2 ist das Diabetes Meßprogramm ausgewählt.

Zur Konfiguration zieht der Arzt jeweils die Geräte aus der Reihe der Kommunikationsgeräte 35 bis 39 des Anwenders in die entsprechenden Segmente 30 bis 33, über die er bei Eintreffen einer klassifizierten Nachricht erreichbar sein möchte. Die Konfiguration wird in der Systemzentrale 6 gespeichert.

Bei der gewählten Konfiguration gemäß Figur 2 wird der Arzt bei dringlichsten Fällen per Telefon 22, bei dringlichen Fällen per E-Mail über den Personal Computer 21 und in allen anderen Fällen per Post benachrichtigt.

Der Kommunikationskreis kann monitorprogramm- bzw. krankheitsspezifisch ausgeprägt sein. In diesem Fall werden für einzelne Überwachungsprogramme die Wege festgelegt, über die der Arzt informiert wird. Dann können dringende Zuckerkrankheitsmeßwerte anders behandelt werden als die dringenden Blutdruckwerte. Welches der Monitorprogramme zur Konfiguration ausgewählt wurde, ist in der Mitte 34 des Kommunikationskreises angegeben.

Bei allgemeiner Ausprägung werden Nachrichten unterschiedlicher Meßprogramme nur anhand ihrer Dringlichkeit weitergeleitet, nicht anhand des Meßwerteprogrammes.

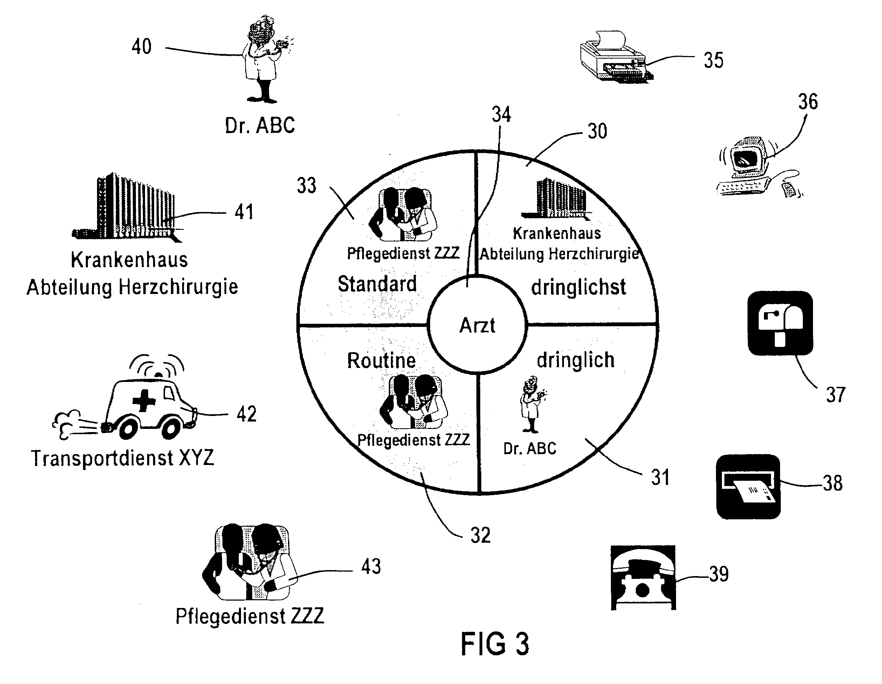

Wenn ein Arzt nicht auf einen Alarm angemessen reagiert - so müssen beispielsweise dringlichste Alarme zentral ausgeschaltet werden, als Zeichen, daß reagiert worden ist - dann leitet die Alarmvorrichtung der Auswertevorrichtung 12 in der Systemzentrale 6 Alarme weiter. Die Konfiguration an wen die Alarme weitergeleitet werden sollen trifft der Arzt, der ein Monitorprogramm startet. Er verwendet dazu wieder einen in Figur 3 dargestellten Kommunikationskreis, der anhand der Dringlichkeitsstufen segmentiert ist. Er zieht in die entsprechenden Kreissegmente die Ärzte und/oder Institutionen 40 bis 43 hinein, die im Notfall zu alarmieren sind, wenn er selbst nicht reagiert.

Bei der gewählten Konfiguration gemäß Figur 3 wird bei dringlichsten Fällen das Krankenhaus 41, bei dringlichen Fällen ein anderer ausgewählter Arzt 40 und in allen anderen Fällen der Pflegedienst benachrichtigt.

Der Kommunikationskreis kann dadurch monitorprogrammspezifisch ausgeprägt sein, daß beispielsweise bei Diabetes Alarmen spezifisch Diabetologen oder bei Herzinfarktverdacht die Herzchirurgie alarmiert werden. Allgemeine Alarme werden unabhängig vom Meßprogramm an andere Stellen weitergeleitet

Es ist zusätzlich vorgesehen, daß z.B. die Alarmierung des Arztes selbst und die ggf. erforderliche Weiterleitung in einem einzigen Kommunikationskreis erfolgen.

Es ist zusätzlich vorgesehen, daß default-Einstellungen vom Server vorgegeben werden, so daß beim Neueinrichten dem Arzt ein sinnvoller Vorschlag vorgegeben wird, wobei eine Präferenzliste des Arztes berücksichtigt wird.

Weiterhin können unterschiedlich komplexe Alarme generiert werden. Im einfachsten Fall werden nur der Arzt oder seine Vertreter unabhängig vom Meßprogramm informiert. Nur der Arzt oder seine Vertreter werden in Abhängigkeit vom Meßprogramm informiert, so werden beispielsweise nur Diabetes Alarme weitergeleitet. Die Alarme können krankheitsspezifisch an unterschiedliche Stellen weitergeleitet werden. So können Diabetes Alarme an diabetologische Kliniken, Herzinfarkte an herzchirurgische Einrichtungen weitergeleitet werden. Die Alarme veranlassen zusätzlich erforderliche Maßnahmen. Beispielsweise alarmieren sie bei Herzinfarktverdacht den monitorenden Arzt, gleichzeitig die Feuerwehrleitstelle und den Transportdienst von der Notfallkette.

Auch der Informationskanal zum Patienten läßt sich konfigurieren, so daß auch der Patient vom Arzt Nachrichten empfangen kann. Dazu kann vom Patienten ebenfalls ein Kommunikationskreis konfiguriert werden, in den er seine Erreichbarkeitswege einträgt.

Ebenso ist vorgesehen, daß der Patient eine Weiterleitung dringender Nachrichten im Urlaub oder Krankenhausaufenthalt einstellen kann.

Die Auswertevorrichtung 12 erzeugt eine Informationsnachricht und gibt sie an den Kommunikations-Server 14 zum Nachrichtenversand weiter. Der Kommunikations-Server 14 als Routingvorrichtung versendet Nachrichten an einen Empfänger unter Zuhilfenahme einer Unterkomponente Telefonbuch, in dem die Kontaktinformationen zu einem adressierbaren Empfänger bereitgestellt werden.

Die Endgeräte bei den Ärzten dienen zur Wiedergabe und Darstellung der medizinischen Daten sowie der Parametrierung des Alarmgebers, der Benachrichtigungswege für Nachrichtenversand und der Endgeräte für Patient und/oder Pfleger.

Die Alarm-Endgeräte dienen der Ausgabe der vom Nachrichtenversand versandten Nachrichten beim Empfänger. Hierzu können spezielle Ausführung des Endgeräts für Patient und/oder Pfleger dienen wie PDA mit Modem, Telefon mit Sprache oder Tonwahl (DTMF), Meßgerät mit integriertem Modem, TV Set-Top-Box, Handy, WWW-Formular oder Papierformular und WWW-Formular

Das Systemzentrale 6 besitzt eine Schnittstelle zur Kopplung mit einem QS-System, um eine Ergebnisqualität unter real-life-Bedingungen zu erhalten. Weiterhin kann das zentrale System eine Schnittstelle zur Kopplung mit einem Abrechnungsmodul aufweisen.

Spezielle Ausführungen der Alarmvorrichtung können eine Alarmauslösung bei pathologischen oder fehlerhaft erfaßten Meßwerten, eine Alarmauslösung bei Compliance-Problemen des Patienten, eine Alarmauslösung bei zu langer Reaktionszeit des Arztes auf dringende Nachrichten und eine Alarmweiterleitung sein, falls Arzt nicht reagiert.

Nachrichten können beispielsweise über Sprachausgabe per Telefon, SMS, E-Mail, WWW, WAP, Fax, Proprietärer Dienst oder Briefpost übermittelt werden.

Die Auswertevorrichtung 12 für die Meßwerte und der Patientendaten-Server 13 können zur Datenerhebung so ausgeführt sein, daß die medizinischen Daten sicher und pseudonymisiert gespeichert werden.

Die Endgeräte für Arzte weisen eine auf Internet-Technologien basierende grafische Benutzeroberfläche an. Zur Parametrierung des Alarmgebers bietet das Gerät das in Figur 2 visualisierte Verfahren an. Zur Parametrierung der Benachrichtigungswege dient das anhand Figur 3 dargestellte Verfahren.

Das Alarm-Endgerät kann je nach Alarmstufe beispielsweise ein Telefon, Handy, E-Mail-Terminal, WWW-Terminal, Fax-Gerät, Terminal zur Nutzung eines proprietären Diensts oder auch der Briefkasten sein.

An der Systemzentrale 6 sind Empfangsgeräte wie beispielsweise ein Faxgerät 20, Personal Computer 21 oder ein Telefon 22 oder Handy angeschlossen, die zu einer Ärzte-Bereitschaft gehören und beispielsweise in einer Gemeinschaftspraxis 23 von Ärzten angeordnet sein.

Das Alarm-Endgerät in der Gemeinschaftspraxis 23 kann einen Rückkanal 29 zur Übertragung von Therapie-Hinweisen an den Patient und/oder Pfleger aufweisen, so daß eine Therapie trotz räumlicher Trennung von Arzt und Patient durchgeführt werden kann (Teletherapie).

Durch das erfindungsgemäße System wird eine "virtuelle" Krankenstation beim Patienten zu Hause geschaffen, die ein zentrales Alarmgebersystem aufweist, das Alarme generiert bei:
- Mangelhafter Patientencompliance (erwartete Meßwerte des Patienten bleiben aus)
- Überschreiten von Schwellwerten (d.h. die Meßwerte liegen in einem krankhaften Bereich)
- Mangelhafter Arztkompliance (erwartete Reaktion auf Alarm durch den Arzt bleibt aus).

Ein Konfigurationskonzept für die Erreichbarkeit und die Alarmweiterleitung ermöglicht die Einstellungen nach den aktuellen Bedürfnisssen jedes Betreuungsfalles.
- Eskalatationsmechanismen sorgen bei fehlender Reaktion eines Arztes für eine Weiterleitung.
- Es lassen sich krankheitsspezifische Alarmweiterleitungen einstellen.
- Es kann eine Behandlungskette wie Notarzt, Transport, Klinik, Pflegedienst und/oder Reha alarmiert und koordiniert werden.
- Eine Konfiguration der Datenkommunikation vom Arzt und/oder Pfleger zum Patienten kann durch den Patienten erreicht werden.

## Patentansprüche

1. Medizinisches System zur Überwachung medizinischer Parameter eines Patienten in häuslicher Umgebung, am Arbeitsplatz oder in Pflege- und Altenheimen mit einer Vorrichtung (2) zur Meßwerterfassung bei dem Patienten, einer Vorrichtung (3 bis 8) zur Übertragung der Meßwerte an eine Systemzentrale (6), mit einer Vorrichtung (24) zur Abfrage der Meßwerte und einer Empfangsvorrichtung (20 bis 22) bei der überwachenden Person, wobei die Systemzentrale (6) einen Speicher (15) für die Meßwerte, eine Auswertevorrichtung (12) für die Meßwerte mit einem Komparator zum Vergleich der Meßwerte mit gespeicherten Sollwerten und mit einer Alarmvorrichtung zur Erzeugung eines Alarmsignals und eine Routingvorrichtung (14) zur Weiterleitung des Alarmsignals an eine Empfangsvorrichtung (3 bis 5, 20 bis 22) der zu alarmierenden Person einer Prozeßkette aufweist.

2. Medizinisches System nach Anspruch 1, **dadurch gekennzeichnet**, daß die Alarmvorrichtung derart ausgebildet ist, daß sie ein Alarmsignal bei Überschreitung der Meßwerte von gespeicherten Sollwertgrenzen erzeugt, das an eine Empfangsvorrichtung (20 bis 22) einer Person einer Behandlungskette geleitet wird.

3. Medizinisches System nach Anspruch 1 oder 2, **dadurch gekennzeichnet**, daß die Alarmvorrichtung ein Alarmsignal bei signifikanter Überschreitung der Meßwerte von gespeicherten Sollwertgrenzen erzeugt, das an eine Empfangsvorrichtung (20 bis 22) einer Person einer Notfallkette geleitet wird.

4. Medizinisches System nach einem der Ansprüche 1 bis 3, **dadurch gekennzeichnet**, daß die Alarmvorrichtung derart ausgebildet ist, daß sie ein Alarmsignal bei Ausbleiben von Meßwerten erzeugt, das an eine Empfangsvorrichtung (3 bis 5) des Patienten geleitet wird.

5. Medizinisches System nach einem der Ansprüche 1 bis 4, **dadurch gekennzeichnet**, daß die Alarmvorrichtung derart ausgebildet ist, daß sie ein Alarmsignal bei Ausbleiben von Reaktionen der Behandlungskette auf besonders gekennzeichnete Werte erzeugt, das an eine Empfangsvorrichtung (20 bis 22) der Notfallkette geleitet wird.

6. Medizinisches System nach einem der Ansprüche 1 bis 5, **dadurch gekennzeichnet**, daß die Routingvorrichtung (14) derart ausgebildet ist, daß das Alarmsignal an eine vorbestimmte, auswählbare Empfangsvorrichtung (3 bis 5, 20 bis 22) der Prozeßkette geleitet wird.

7. Medizinisches System nach einem der Ansprüche 1 bis 6, **dadurch gekennzeichnet**, daß die Routingvorrichtung (14) derart ausgebildet ist, daß das Alarmsignal durch automatisches Routing an eine von der Routingvorrichtung (14) unter Berücksichtigung der Verfügbarkeit bestimmte Empfangsvorrichtung (3 bis 5, 20 bis 22) der Prozeßkette geleitet wird.

8. Medizinisches System nach einem der Ansprüche 1 bis 7, **dadurch gekennzeichnet**, daß die Routingvorrichtung (14) ein lernendes Expertensystem aufweist, das die Alarmierung der Prozeßkette bewirkt.

9. Medizinisches System nach einem der Ansprüche 1 bis 8, **dadurch gekennzeichnet**, daß die Auswertevorrichtung (12) ein lernendes Expertensystem aufweist, das die Meßwerte krankheits- und/oder problemspezifisch anhand von Regelsystemen oder auf Wahrscheinlichkeiten basierend interpretiert, aufgrund deren Ergebnis das Alarmsignal ausgelöst wird.

10. Medizinisches System nach einem der Ansprüche 1 bis 9, **dadurch gekennzeichnet**, daß die Alarmvorrichtung derart ausgebildet ist, daß sie in Abhängigkeit von der Dringlichkeit einer Reaktion unterschiedliche Alarmsignale erzeugt, die an Empfangsvorrichtungen (3 bis 5) der Behandlungs- und/oder Notfallkette geleitet wird
